(19) Europäisches Patentamt
European Patent Office
Office européen des brevets

(11) Veröffentlichungsnummer: 0 312 735
A2

(12) # EUROPÄISCHE PATENTANMELDUNG

(21) Anmeldenummer: 88113767.3

(51) Int. Cl.4: C07C 49/603 , C07C 45/67

(22) Anmeldetag: 24.08.88

(30) Priorität: 17.10.87 DE 3735211

(43) Veröffentlichungstag der Anmeldung:
26.04.89 Patentblatt 89/17

(84) Benannte Vertragsstaaten:
AT BE CH DE FR GB IT LI LU NL SE

(71) Anmelder: HÜLS AKTIENGESELLSCHAFT
- RSP Patente / PB 15 - Postfach 13 20
D-4370 Marl 1(DE)

(72) Erfinder: Bellut, Hans, Dr.
Bergstrasse 50
D-4408 Dülmen(DE)

(54) **Verfahren zur Herstellung von beta-Isophoron aus alpha-Isophoron.**

(57) Die Umlagerung von beta-Isophoron aus alpha-Isophoron kann mit Methylenmagnesiumjodid und Eisen(III)-chlorid, ferner mit Triäthanolamin oder mit organischen Säuren, z. B. Adipinsäure, erfolgen. Nachteilig sind bei diesem Verfahren ein hoher Chemikalienverbrauch mit Aufarbeitungs- und Entsorgungsproblemen, die Ausbeuten sind ungünstig.

Die Verwendung von Acetylacetonaten von Übergangsmetallverbindungen bzw. von Al führt zu einer beträchtlichen Ausbeutesteigerung, verbunden mit einer erheblichen Reduzierung der Katalysatormenge.
beta-Isophoron findet Anwendung insbesondere in der Riechstoffindustrie und für Vitaminsynthesen.

EP 0 312 735 A2

## Verfahren zur Herstellung von beta-Isophoron aus alpha-Isophoron

beta-Isophoron besitzt großes wirtschaftliches Interesse als Grundstoff für organische Präparate, z. B. in der Riechstoffindustrie, sowie als Ausgangsprodukt für verschiedene Vitaminsynthesen. Bei der wasserabspaltenden Trimerisation von Aceton fällt jedoch hauptsächlich das alpha-Isomere in über 90 % an. Eine möglichst quantitative, einfache Umwandlung ist deshalb von besonderem Interesse, da aus dem beta-Isomeren als Grundstoff naturidentische Produkte aufgebaut werden können, die wegen ihrer Wirkungsweise und ihres Abbauverhaltens wenig bis keine Probleme aufwerfen.

Die Verschiebung der Doppelbindung in dem bei der Synthese anfallenden Isophoron bereitet deswegen große Schwierigkeiten, weil

1. die Doppelbindung aus der Konjugation zur Carbonylgruppe verschoben werden muß,
2. das System auf stärkere alkalische oder auch saure Katalysatoren leicht mit Wasserabspaltung und Verharzung reagiert,
3. der Energieinhalt des Moleküls deutlich erhöht werden muß und
4. die Einstellung des Gleichgewichts nur langsam erfolgt.

Wegen des relativ geringen Anteils an beta-Isophoron im Gleichgewicht bereitet die kontinuierliche Entfernung des gewünschten Isomeren erhebliche technische Schwierigkeiten; sie ist jedoch in jedem Fall Voraussetzung für ein praktikables Verfahren. Da an der Gleichgewichtslage aus allgemein bekannten thermodynamischen Gründen nichts zu ändern ist, konnte nur versucht werden, die Geschwindigkeit der Einstellung zu beeinflussen, ohne dabei jedoch Nebenreaktionen mehr als tragbar zu begünstigen.

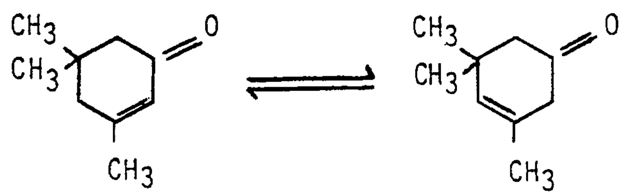

alpha-Isophoron beta-Isophoron

Es sind im Laufe der Zeit eine Reihe von Vorschlägen zur Bereitung von beta-Isophoron bekannt geworden, die jedoch alle erhebliche Nachteile aufweisen, so daß bis jetzt noch keine dieser Vorschläge in die Praxis bzw. in technischem Maßstab umgesetzt werden konnte. Eine Umlagerung wird z. B. erreicht durch Umsetzung molarer Mengen alpha-Isophoron mit Methylmagnesiumjodid unter Zusatz von Eisen(III)-chlorid, anschließender Hydrolyse und destillativer Aufarbeitung (A. Heymes und P. Teisseire, Recherches 1971, 18, 104-8). Die Isomerisierung kann auch durch mehrstündiges Kochen mit Triäthanolamin, anschließender Fraktionierung und Waschen des Destillats mit Weinsäure und Kochsalzlösung erfolgen (Firmenich S.A., DE-OS 24 57 157). Durch Katalyse wenig dissoziierter organischer Säuren wird die Umsetzung ebenfalls erreicht. In einem Verfahren werden bei Verwendung von Adipinsäure ca. 70 % Ausbeute reinen beta-Isophorons erreicht (Hoffmann-La Roche + Co. AG, DE-PS 25 08 779).

Alle bekannt gewordenen Verfahren weisen den Nachteil auf, daß entweder ein hoher Chemikalienverbrauch mit entsprechenden Aufarbeitungs-und Entsorgungsproblemen in Kauf genommen werden muß, oder daß die verwendeten Katalysatoren zu schwach basisch bzw. sauer sind, um eine tragbare Raum/Zeit-Ausbeute zu erzielen. Eine deutliche Erhöhung bzw. Erniedrigung der Acidität der Katalysatoren verbietet sich, da sonst verstärkt Verharzungen und Wasserabspaltung durch Selbstkondensation des Isophorons erfolgen.

Es war daher Aufgabe der Erfindung, ein Verfahren zur Gewinnung von beta-Isophoron aus alpha-Isophoron zu finden, das die Nachteile des Standes der Technik nicht aufweist, technisch wenig aufwendig ist und zu guten Ausbeuten führt.

Die Lösung des Problems gelang schließlich durch den Einsatz komplex gebundener Übergangsmetalle als Katalysatoren.

Gegenstand der Erfindung ist daher ein Verfahren zur Herstellung von beta-Isophoron aus alpha-Isophoron, welches dadurch gekennzeichnet ist, daß alpha-Isophoron in Gegenwart von Acetylacetonaten der Metalle der Gruppen IVB, VB, VIB, VIIB und VIIIB des Periodensystems sowie von Aluminium destillativ

behandelt wird.

Die Gruppeneinteilung erfolgt nach der Bezeichnung in Chemical Abstracts.

Besonders geeignete Katalysatoren sind die Acetylacetonate von Fe, Co, Cr, Mn und Al.

Durch die Komplexbildung wird die Agressivität, wie sie bei Friedel-Crafts-Verbindungen, wie beispielsweise bei $AlCl_3$, $FeCl_3$ usw. bekannt ist, stark herabgesetzt. Jedoch bleibt die Isomerisierungsfähigkeit für Doppelbindungen in empfindlichen Systemen offenbar zumindest zum Teil erhalten.

Es stellte sich bei Versuchen heraus, daß allgemein insbesondere Übergangsmetallacetylacetonate sich besonders gut als Verschiebungskatalysatoren eignen. Obwohl die Metallatome sehr fest in diesen Komplexen eingebunden sind, können sie offensichtlich die Einstellung des Gleichgewichts erheblich beschleunigen. Es werden zur Isomerisierung 0,01 bis 10 % Gewichteile, insbesondere 0,1 bis 1,0 % Gewichtsteile, benötigt. Die erhebliche Reduktion an Katalysatormenge muß als bedeutsame Verfahrensverbesserung gesehen werden. Außerdem wird die Reaktionszeit um wenigstens 50 % verringert, was bei vergleichbarer Auslegung der Apparaturen zu einer Verdoppelung der Raum/Zeit-Ausbeute führt.

Das hier beschriebene Verfahren gestaltet sich technisch relativ einfach und kann kontinuierlich und diskontinuierlich durchgeführt werden.

Zu einer mit alpha-Isophoron und 0,1 bis 1 % mit einem Übergangsmetallacetylacetonat beschickten Destillationsblase wird kontinuierlich frisches technisches Isophoron dosiert. Die Zugabe erfolgt in der Menge, wie am Kopf einer gut trennenden Fraktionierkolonne beta-Isophoron abgezogen wird. Das auf diese Weise erhaltene beta-Isophoron besitzt bereits eine Reinheit von 95 % und kann durch erneute Fraktionierung beliebig aufkonzentriert werden. Die benötigte Isomerisierungsenergie wird aus der Blasenheizung der bei Normaldruck arbeitenden Apparatur gedeckt. Es stellt sich bei dieser Arbeitsweise eine Kopftemperatur von 187 °C ein. Dieses Verfahren arbeitet trotz des erzielten Fortschritts nicht völlig verlustfrei; ca. 5 bis 7 % des durchgesetzten Isophorons fällt im Sumpf als Überkondensat an und muß verworfen werden. Es kann jedoch mit einer Ausbeute von 90 bis 95 % beta-Isophoron, bezogen auf umgesetztes Einsatzprodukt, gerechnet werden.

Es empfiehlt sich, bei Normaldruck zu arbeiten, um die Siededifferenz zwischen alpha- und beta-Isophoron auszunutzen. Beim Arbeiten bei vermindertem Druck erhöhen sich die Ansprüche an die Trennleistung der Kolonne.

## Beispiel 1

Aus einem 1 l-Kolben werden 500 ml technisches Isophoron (GC-Analyse: 98,7 % alpha-Isophoron, 0,98 % beta-Isophoron) nach Versetzen mit 2 g Eisen(III)acetylacetonat (entsprechend 0,43 Gew.-%) über eine Fraktionierkolonne abdestilliert. Es genügt eine 1 m Kolonne mit 2,5 cm Durchmesser und einer Füllung aus "Multifil" V4A, deren Vorheizung auf 155 °C eingestellt ist. Die benötigte Sumpftemperatur beträgt zu Anfang 215 °C und muß gegen Ende auf 250 °C gesteigert werden. Als Kopftemperatur stellt sich, sobald die Apparatur im Gleichgewicht ist, ein Wert von 186 °C ein. Das System bleibt bei einem Abnahme/Rücklaufverhältnis von 1 : 10 stabil, und es lassen sich bei der beschriebenen Dimensionierung 20 ml Produkt pro Stunde abnehmen.

Es werden 445 g, entsprechend 89 % Rohausbeute (GC-Analyse: 94 % beta-Isophoron, 5 % alpha-Isophoron), erhalten neben 55 g (11 Gew.-%) Rück stand. Der Rückstand besteht erfahrungsgemäß (vgl. Beispiel 4) zu 70 bis 80 % aus alpha-Isophoron und kann nach Aufarbeitung zurückgeführt werden.

Durch einfache Destillation über eine 40 cm Vigreux Kolonne bei einer Abnahme von 100 ml/h wird 99 %iges beta-Isophoron aus dem Rohprodukt erhalten.

Sofern das zurückgewinnbare alpha-Isophoron berücksichtigt wird, wird bei dieser Arbeitsweise eine Reinausbeute von 97 bis 98 % an beta-Isophoron erzielt.

## Beispiele 2 bis 9

In gleicher Weise wie in Beispiel 1 werden Isomerisierungen mit verschiedenen Katalysatoren unternommen. Die Ergebnisse sind in der folgenden Tabelle zusammengefaßt:

| Beispiel Nr. | Katalysator-Metall | Katalysator % | Produktgewinng. ml/h | Roh-Ausbeute % | Reinausb. bez. auf Umsatz | Rückstand % |
|---|---|---|---|---|---|---|
| 2 | Fe(III) | 0,44 | 20 | 80 | 95,4 | 20 [a] |
| 3 | Co(III) | 1,0 | 20 | 77 | 93,9 | 22 [a] |
| 4 | Co(III) | 1,0 | 20 | 77 | 94,7 | 23 [a] |
| 5 | Cr(III) | 1,0 | 10 | 74 | 93,3 | 25 [a] |
| 6 | Al(III) | 1,0 | 10-15 | 77 | 94,7 | 23 [a] |
| 7 | Ni(II)[b] | 1,0 | 10 | 48 | - | - |
| 8 | Mn(II) | 1,0 | 20-10 | 73 | 93,0 | 26 [a] |
| 9 | Ti(IV)[b] | 1,0 | 20 | 44 | - | - |

a) Der Rückstand besteht zu 77 % (GC-Analyse) aus alpha-Isophoron, das rückgeführt werden kann
b) Der Katalysator verliert im Laufe des Versuchs seine Aktivität

Beispiel 10

Analog Beispiel 1 werden 1 800 g alpha-Isophoron an 0,11 Gew.-% Eisen(III)acetylacetonat umgesetzt, wobei laufend das abdestillierte Rohprodukt durch frisches Einsatzprodukt ersetzt wird. Es wird eine Rohausbeute von 84 % erzielt und 15 % als Rückstand zurückbehalten.

## Ansprüche

Verfahren zur Herstellung von beta-Isophoron aus alpha-Isophoron, dadurch gekennzeichnet, daß alpha-Isophoron in Gegenwart von Acetylacetonaten der Metalle der Gruppen IVB, VB, VIB, VIIB und VIIIB des Periodensystems sowie von Aluminium destillativ behandelt wird.